Europäisches Patentamt

European Patent Office

Office européen des brevets

(19)

(11) Veröffentlichungsnummer: **0 268 826**
**B1**

## (12) EUROPÄISCHE PATENTSCHRIFT

(45) Veröffentlichungstag der Patentschrift:
28.02.90

(21) Anmeldenummer: 87115415.9

(22) Anmeldetag: 21.10.87

(51) Int. Cl.⁴: **C07C 29/132**, C07C 35/08,
C07C 29/88

(54) Verfahren zur Aufbereitung von Cyclohexylhydroperoxid enthaltenden Reaktionsgemischen.

(30) Priorität: 23.10.86 DE 3636056
08.01.87 DE 3700336

(43) Veröffentlichungstag der Anmeldung:
01.06.88 Patentblatt 88/22

(45) Bekanntmachung des Hinweises auf die Patenterteilung:
28.02.90 Patentblatt 90/9

(84) Benannte Vertragsstaaten:
BE CH DE ES FR GB IT LI NL

(56) Entgegenhaltungen:
US-A- 3 351 635
US-A- 3 526 645
US-A- 4 465 861

(73) Patentinhaber: BASF Aktiengesellschaft,
Carl-Bosch-Strasse 38, D-6700 Ludwigshafen(DE)

(72) Erfinder: Neubauer, Gerald, Dr., Mozartstrasse 24,
D-6940 Weinheim(DE)
Erfinder: Schnabel, Rolf, Dr., Frankenstrasse 22,
D-6707 Schifferstadt(DE)
Erfinder: Hartig, Juergen, Dr., In der Schleit 9,
D-6718 Gruenstadt(DE)
Erfinder: Ritz, Josef, Dr., Osloer Weg 8,
D-6700 Ludwigshafen(DE)

## Beschreibung

Aus der EP-A-92 867 ist bekannt, daß man Cyclohexylhydroperoxid enthaltende Reaktionsgemische aus der Cyclohexan-Oxidation mit wäßrigen Lösungen von Alkalimetallhydroxiden, die gegebenenfalls katalytisch wirksame Metallsalze enthalten können, behandelt um das im Reaktionsgemisch enthaltene Cyclohexylhydroperoxid in Cyclohexanol und Cyclohexanon umzuwandeln. Dieses Verfahren hat den Nachteil, daß man erhebliche Mengen an Alkalimetallhydroxiden anwenden muß, die zu entsorgen sind und zudem beträchtliche Mengen an Cyclohexanon und/oder Cyclohexanol unter Bildung von Hochsiedern verloren gehen.

Nach einem anderen aus der CH-PS 545 758 bekannten Verfahren werden Cyclohexylhydroperoxid enthaltende Reaktionsgemische aus der Cyclohexan-Oxidation zunächst in Gegenwart eines Edelmetallkatalysators hydriert und anschließend das so erhaltene Hydriergemisch mit wäßrigen Alkalilaugen behandelt. Dieses Verfahren hat den Nachteil, daß ebenfalls entsorgungsbedürftige Ablaugen anfallen und sich erhebliche Mengen an hochsiedenden Nebenprodukten bilden. Darüber hinaus erhält man je Mol Cyclohexylhydroperoxid lediglich ein Mol Cyclohexanol.

Es wurden auch schon Trägerkatalysatoren für die Zersetzung von Cyclohexylhydroperoxid verwendet. In der US-PS 28 51 496 werden Metalle der 8. Gruppe, z.B. Kobalt auf aktiviertem Aluminiumoxid als geeignete Katalysatoren beschrieben. Diese Katalysatoren haben jedoch den Nachteil, daß sie wasser- und säureempfindlich sind und ihre Lebensdauer deshalb sehr beschränkt ist. Zudem läßt die Ausbeute an Wertprodukten zu wünschen übrig.

Aus der SU-Patentschrift 739 051 ist ein Verfahren bekannt, bei dem man Cyclohexylhydroperoxid enthaltende Reaktionsgemische mit einem Cycloolefin wie Cyclooctadien oder Cyclododecatrien umsetzt und einerseits Cyclohexanol und andererseits Cyclooctadienepoxid oder Cyclododecatrienepoxid gewinnt. Das Verfahren hat den Nachteil, daß man je Mol Cyclohexylhydroperoxid lediglich ein Mol Cyclohexanol erhält.

Ferner ist aus J. org. Chem. Band 45, Seiten 4139 bis 4143 bekannt, daß man Cyclohexenoxid zu Cyclohexanol hydriert. Ein Hinweis wie Cyclohexylhydroperoxid enthaltende Reaktionsgemische zu behandeln sind wird nicht gegeben.

Aus der US-A-3 526 64 ist ein Verfahren zur Herstellung von Epoxiden bekannt, bei dem man Hydroperoxide u.a. Cyclohexenylhydroperoxid mit Olefinen u.a. Cyclohexen in Gegenwart von Zweikomponentenkatalysatoren aus Metallen der V. und VI. Gruppe einerseits und der VIII. Gruppe andererseits umsetzt. In der Technik hat man von diesem Verfahren für die Aufarbeitung von Cyclohexanoxidationsgemischen bisher abgesehen, da solche Gemische eine Vielzahl von Nebenprodukten enthalten und somit mit der Bildung von unbekannten störenden Verbindungen gerechnet wurde.

Entsprechend dem in der US-A-3 351 635 beschriebenen Verfahren erhält man Epoxide durch Umsetzen von Olefinen u.a. Cyclohexen mit einem peroxidierten Gemisch aus Cyclohexanon und Cyclohexanol in Gegenwart von Metallverbindungen z.B. von Molybdän oder Wolfram. Hierfür gelten die vorgenannten Bedenken, zumal von Cyclohexanon eine Vielzahl von Peroxiden bekannt ist.

Es war die technische Aufgabe gestellt ein Verfahren zur Aufarbeitung von Cyclohexylhydroperoxid enthaltenden Reaktionsgemischen zur Verfügung zu stellen, bei dem die Ausbeute an Cyclohexanol erhöht wird und möglichst geringe Mengen an hochsiedenden Nebenprodukten sowie keine behandlungsbedürftige Ablaugen anfallen.

Diese Aufgabe wird gelöst in einem Verfahren zur Aufarbeitung von Cyclohexylhydroperoxid enthaltenden Reaktionsgemischen, die durch Oxidation von Cyclohexan mit molekularem Sauerstoff oder solchen enthaltenden Gasen in flüssiger Phase bei einer Temperatur von 130°C bis 200°C und unter einem Druck von 5 bis 125 bar erhalten worden sind, durch Umsetzen mit Cycloolefinen bei erhöhter Temperatur in Gegenwart von Katalysatoren, dadurch gekennzeichnet, daß man Cyclohexylhydroperoxid mit Cyclohexen bei erhöhter Temperatur in Gegenwart von in Cyclohexan löslichen Übergangsmetallverbindungen der 4. bis 6. Gruppe des periodischen Systems oder mindestens einer in Cyclohexan unlöslichen Verbindung eines Übergangsmetalls der 4. bis 6. Gruppe, Selen, Tellur oder einem Borid umsetzt und dann das entstandene Cyclohexenoxid in Gegenwart von Hydrierkatalysatoren bei erhöhter Temperatur in an sich bekannter Weise zu Cyclohexanol hydriert.

Das neue Verfahren hat den Vorteil, daß keine behandlungsbedürftigen Ablaugen anfallen und der Anteil an hochsiedenden Nebenverbindungen vermindert wird. Weiter hat das Verfahren den Vorteil, daß es im wesentlichen unter Bildung von Cyclohexanol verläuft und insbesondere daß je Mol Cyclohexylhydroperoxid bis zu 2 Mol Cyclohexanol erzeugt werden und somit mehr Wertprodukte je Mol oxidiertes Cyclohexan erzeugt werden.

Erfindungsgemäß geht man von Cyclohexylhydroperoxid enthaltenden Reaktionsgemischen aus, die durch Oxidation von Cyclohexan mit molekularem Sauerstoff oder solchen enthaltenden Gasen, z.B. Luft in flüssiger Phase erhalten worden sind. Hierbei hält man eine Temperatur von 130 bis 200°C und einen Druck von 5 bis 125 bar ein. Gegebenenfalls werden Katalysatoren wie Kobaltsalze mitverwendet. Vorteilhaft führt man die Oxidation bis zu einem Umsatz von 2 bis 8 Gew.%, bezogen auf eingesetztes Cyclohexan durch. Druck und Temperatur werden so aufeinander abgestimmt, daß zu jedem Zeitpunkt die Umsetzung in flüssiger Phase erfolgt.

Nach einem geeigneten Verfahren führt man die Oxidation von Cyclohexan z.B. in einer senkrecht stehenden Reaktionszone durch, die durch Lochbleche, die in gleichmäßigen Abständen angeordnet sind, in Kammern unterteilt ist. Die Lochbleche haben vorteilhaft einen freien Querschnitt von 3 bis 20 %. Oberhalb jeden Lochblechs sind über den Querschnitt gleichmäßig verteilt Düsen angeordnet, wobei die Zuführungsöffnungen mit Erweiterungen versehen sind, deren Öffnung nach unten zeigt. Durch die Reaktionszone leitet man von unten nach oben Cyclohexan. Gleichzeitig führt man molekularen Sauerstoff enthaltende Gase, z.B. mit einem Sauerstoffgehalt von 5 bis 30 Vol.%, vorteilhaft Luft durch jede Düsenöffnung zu, so daß Gasblasen von 10 bis 50 mm Durchmesser entstehen, die im Verlauf der Reaktionszone nach oben in kleinere Gasblasen, d.h. Gleichgewichtsblasen zerfallen. Die Menge an zugeführten molekularen Sauerstoff enthaltenden Gasen und Cyclohexan wird so aufeinander abgestimmt, daß das aus der Reaktionszone austretende Abgas einen Gehalt an molekularem Sauerstoff von nicht mehr als 0,1 bis 1,5 Vol.% enthält.

Nach einem besonders bevorzugten Verfahren wird in einer ersten Stufe molekularer Sauerstoff in Cyclohexan durch in Berührung bringen mit molekularem Sauerstoff oder solchen enthaltenden Gasen bei einer Temperatur von 10 bis 50°C unter erhöhtem Druck, z.B. von 40 bis 100 bar gelöst und dann in einer zweiten Stufe das gelösten molekularen Sauerstoff enthaltende Cyclohexan bei einer Temperatur von 130 bis 250°C und vorteilhaft unter einem mindestens 10 bar höheren Druck durch eine rohrförmige Reaktionszone angenähert in Pfropfenströmung ohne Ausbildung einer Gasphase geleitet, wobei die Verweilzeit so bemessen ist, daß sie zwischen der Zeit, zu der 50 % der Menge an gelöstem Sauerstoff verbraucht ist und dem 1,2fachen der Zeit, die zum Verbrauch von 99,9 % des gelösten Sauerstoffs erforderlich ist, liegt. Ein geeignetes Verfahren wird beispielsweise beschrieben in der EP-A-150 821.

Solche Reaktionsgemische können zweckmäßig vor der Weiterverarbeitung durch partielles Abdestillieren von Cyclohexan, z.B. durch Flash-Destillation bei der Entspannung angereichert werden. Typische Reaktionsgemische enthalten beispielsweise neben Cyclohexan 0,5 bis 5,0 Gew.% Cyclohexylhydroperoxid, 0,1 bis 2,5 Gew.% Cyclohexanol sowie 0,1 bis 1,5 Gew.% Cyclohexanon, ferner Nebenprodukte wie Ester, Carbonsäuren sowie gegebenenfalls Wasser bis zu 2 Gew.%.

Vorteilhaft werden solche Reaktionsgemische vor der Weiterbehandlung mit Wasser und/oder wäßrigen Alkalicarbonatlösungen gewaschen.

In einer zweiten Stufe wird Cyclohexylhydroperoxid zweckmäßig in dem Reaktionsgemisch, wie es erhalten worden ist, mit Cyclohexen bei erhöhter Temperatur in Gegenwart von in Cyclohexan löslichen Verbindungen von Übergangsmetallen der 4. bis 6. Gruppe des periodischen Systems oder mindestens einer in Cyclohexan unlöslichen Verbindung eines Übergangsmetalls der 4. bis 6. Gruppe, Selen, Tellur oder einem Borid umgesetzt.

Vorteilhaft gibt man je Mol Cyclohexylhydroperoxid 1 bis 5 Mol, insbesondere 1 bis 2 Mol Cyclohexen zu. Vorteilhaft verwendet man Gemische aus Cyclohexen und Cyclohexan, z.B. mit einem Gehalt von 10 bis 25 Gew.% Cyclohexen, wie sie durch partielle Hydrierung von Benzol in Gegenwart von Rutheniumkatalysatoren erhalten werden. Ein geeignetes Verfahren wird beispielsweise beschrieben in der US-PS 39 12 787. Die Umsetzung wird vorzugsweise bei einer Temperatur von 40 bis 150°C, insbesondere bei einer Temperatur von 60 bis 100°C durchgeführt. Sofern die Temperatur unterhalb dem Siedepunkt des Cyclohexans liegt, wird zweckmäßig Atmosphärendruck angewandt, während bei höheren Temperaturen erhöhter Druck, z.B. bis 10 bar angewandt wird. Druck und Temperatur werden zweckmäßig so aufeinander abgestimmt, daß eine flüssige Phase eingehalten wird.

Die Umsetzung wird in Gegenwart von Übergangsmetallverbindungen der 4. bis 6. Gruppe des periodischen Systems, die in Cyclohexan löslich sind, durchgeführt, z.B. von Verbindungen der Metalle Titan, Zirkon, Vanadium, Niob, Tantal, Chrom, Molybdän oder Wolfram. Geeignete Verbindungen sind beispielsweise Acetylacetonate oder Salze mit höheren Fettsäuren, z.B. mit 8 bis 18 Kohlenstoffatomen wie 2-Ethylhexansäure, Undecansäure, Stearinsäure oder Palmitinsäure, ferner Naphthenate. Besonders bevorzugt sind Verbindungen von Molybdän, Titan, Vanadium und Wolfram. Besonders Bedeutung haben Molybdän-Verbindungen erlangt. Vorteilhaft wendet man je Mol Cyclohexylhydroperoxid 0,1 bis 1,0 mMol der löslichen Katalysatoren, berechnet als Metall an. Die Behandlungsdauer beträgt in der Regel von 20 bis 60 Minuten.

Andere geeignete Katalysatoren sind in Cyclohexan unlösliche Verbindungen von Metallen der 4. bis 6. Nebengruppe der periodischen Systems, z.B. Titan, Zirkon, Vanadium, Niob, Tantal, Chrom, Molybdän oder Wolfram. Besonders geeignet sind deren oxidische Verbindungen wie Titandioxid, Zirkondioxid, Vanadiumoxid, Chromoxide, Molybdänoxid, Molybdate, Wolframoxid oder Wolframate.

Ferner sind geeignete Katalysatoren in Cyclohexan unlösliche, vorzugsweise oxidische Verbindungen von Selen oder Telur, wie Tellurdioxid, tellurige Säure, Selendioxid oder selenige Säure.

Geeignete Katalysatoren sind auch Boride wie Zinkborid, Chromborid oder Calciumborid.

Solche in Cyclohexan unlöslichen Katalysatoren werden vorteilhaft in Mengen von 0,01 bis 5 Gew.% bezogen auf Cyclohexylhydroperoxid angewandt.

Die Katalysatoren können in Suspension vorteilhaft jedoch in fest angeordneter Form angewandt werden. Es hat sich hierbei besonders bewährt, die Katalysatoren in Form von Trägerkatalysatoren zu verwenden. Geeignete Träger sind beispielsweise Aktivkohle, Alumniumoxid, Siliciumdioxid oder Silicagel, ferner Aluminiumsilikate, insbesondere Zeolithe. Der Gehalt an aktiven Metallverbindungen auf den Trägerkatalysatoren beträgt vorteilhaft 1 bis 10 Gew.%, berechnet als katalytisch wirksame Verbindung.

Die katalytisch aktiven Metalle können auch an Ionenaustauscher gebunden angewendet werden. Falls Metalle als Kation vorliegen, eignen sich stark saure Ionenaustauscher, z.B. vernetztes Polystyrol mt Sulfonsäuregruppen oder falls die katalytisch aktiven Elemente in saurer Form vorliegen, wie selenige Säure oder Molybdat, eignen sich auch stark basische Ionenaustauscher, beispielsweise vernetztes Polystyrol mit tertiären oder quatrnären Ammoniumgruppen.

Ferner hat es sich bewährt, zusätzlich zu den vorgenannten katalytisch aktiven Elementen basi-

sche Verbindungen aus der Gruppe der Alkali- oder Erdalkaliverbindungen, wie Alkalicarbonate oder Erdalkalioxide, ferner Alkali- oder Erdalkaliphosphate mit zu verwenden. Geeignete Promotoren sind auch Aluminium oder Zinkphosphat.

In einer weiteren Stufe wird das entstandene Cyclohexenoxid zweckmäßig in dem Reaktionsgemisch, in dem es anfällt, in Gegenwart von Hydrierkatalysatoren bei erhöhter Temperatur zu Cyclohexanol hydriert. Vorteilhaft führt man die Hydrierung bei einer Temperatur von 80 bis 150°C, insbesondere von 100 bis 130°C durch. Hierbei hält man zweckmäßig einen Druck von 3 bis 30 bar, insbesondere von 10 bis 20 bar ein.

Geeignete Hydrierkatalysatoren sind z.B. Metalle der 8. Nebengruppe des periodischen Systems wie Kobalt, Nickel, Palladium, Platin oder Ruthenium. Die Katalysatoren können als solche in fein verteilter Form als Suspension oder fest angeordnet, vorteilhaft auf einen Träger wie Aluminiumoxid, Kohle, Magnesiumoxid, Aluminiumsilikat oder Siliciumdioxid niedergeschlagen angewandt werden. Besonders bewährt haben sich Palladiumkatalysatoren, insbesondere Palladium auf Aktivkohle, vorteilhaft mit einer Palladiumkonzentration von 0,1 bis 10 Gew.%, insbesondere 1 bis 5 Gew.%. Es hat sich auch bewährt, wenn ein solcher Katalysator fest angeordnet in Sumpf- oder Rieselfahrweise angewandt wird. Bei der Hydrierung wird überschüssiges Cyclohexen zu Cyclohexan hydriert, das mit dem als Lösungsmittel vorhandenen Cyclohexan abgetrennt und wieder der Oxidationsstufe zugeführt wird. Gegebenenfalls noch vorhandenes nicht umgesetztes Cyclohexylhydroperoxid wird zu Cyclohexanol hydriert.

Aus dem Reaktionsgemisch wird Cyclohexanol, und das ebenfalls vorhandene Cyclohexanon durch Destillation abgetrennt.

Das nach dem Verfahren der Erfindung erhältliche Cyclohexanol eignet sich zur Herstellung von Adipinsäure, einem wichtigen Faserrohstoff.

Das Verfahren nach der Erfindung sei an folgenden Beispielen veranschaulicht.

## Beispiel 1

In einem Reaktionsgefäß werden 300 l einer Lösung mit 85,4 mMol.% Cyclohexen, 85,7 mMol.% Cyclohexylhydroperoxid, 36,9 mMol.% Cyclohexanol, 18,4 mMol.% Cyclohexanon und 0,1 mMol.% $MoO_2$-Ethylhexanoat in Cyclohexan 3 Stunden auf eine Temperatur von 80°C erhitzt. Nach gaschromatographischer Analyse erhält die Lösung nach dem Abkühlen 19,6 mMol.% Cyclohexen, 4,6 mMol.% Cyclohexylhydroperoxid, 107,9 mMol.% Cyclohexanol, 26,7 mMol.% Cyclohexanon und 65,8 mMol.% Cyclohexenoxid.

## Beispiel 2

Man verfährt wie in Beispiel 1 beschrieben, verwendet jedoch eine Lösung, die 240,6 mMol.% Cyclohexen, 77,5 mMol.% Cyclohexylhydroperoxid, 48,7 mMol.% Cyclohexanol und 34 mMol.% Cyclohexanon in Cyclohexan enthält. Die Katalysatorkonzentration beträgt 0,1 mMol.%. Der Austrag erhält nach der Reaktion 165,0 mMol.% Cyclohexen, 0,8 mMol.% Cyclohexylhydroperoxid, 120,6 mMol.% Cyclohexanol, 35,2 mMol.% Cyclohexanon und 75,5 mMol.% Cyclohexenoxid.

## Beispiel 3

Ein Reaktionsrohr mit 90 cm² Querschnitt wird mit 36,5 kg eines 5 gew.%igen Palladium-Aktivkohlekatalysators gefüllt. Durch dieses Rohr wird bei 100°C und unter einem Druck von 10 bar der in Beispiel 1 erhaltene Reaktionsaustrag 3 Stunden im Kreislauf gepumpt. Der Mengenstrom beträgt 7,5 l/min. Gleichzeitig werden 450 N Liter/min Wasserstoff von unten durch das Reaktionsrohr geleitet. Aus dem Abgas werden durch Kühlen die dampfförmig erhaltenen Produkte ausgeschieden und in den Kreislauf zurückgeführt. Nach Beendigung der Reaktion ergibt eine gaschromatographische Analyse einen Gehalt von 176,4 mMol.% Cyclohexanol, 26,7 mMol.% Cyclohexanon. Cyclohexen und Cyclohexylhydroperoxid sowie Cyclohexenoxid sind nicht nachweisbar. Der Gehalt an Schwersiedern beträgt 0,2 Gew.%. Dies entspricht einem Verlust von jeweils 1,0 mMol.% Cyclohexenoxid und Cyclohexanol durch Veretherung beider Verbindungen.

## Beispiel 4

a) Herstellung des Katalysators

Ein stark saures Ionenaustauscherharz auf der Basis Styrol/Divinylbenzol mit Sulfonsäuregruppen wird mit einer 10 gew.%igen wäßrigen Titansulfatlösung getränkt. Anschließend wäscht man den mit etwa 5 Gew.% Titan beladenen Ionenaustauscher mit Wasser aus und neutralisiert die restlichen sauren Gruppen mit einer wäßrigen Natriumbicarbonatlösung. Nach erneutem Auswaschen mit Wasser wird der Katalysator getrocknet.

b) Umsetzung von Cyclohexylhydroperoxid mit Cyclohexen

3 l einer Lösung werden mit 85,4 mmol.% Cyclohexen, 85,7 mmol.% Cyclohexylhydroperoxid, 36,9 mmol.% Cyclohexanol, 18,4 mmol.% Cyclohexanon in Cyclohexan mit 360 g des oben genannten Katalysators 3 Stunden auf eine Temperatur von 80°C erhitzt. Nach gaschromatographischer Analyse erzielt man eine Ausbeute von 98 % Cyclohexenoxid.

## Beispiel 5

In einem Reaktionsgefäß werden 3 l einer Lösung mit 85,4 mmol.% Cyclohexen, 85 mmol.% Cyclohexylhydroperoxid, 36,9 mmol.% Cyclohexanol, 18,4 mmol.% Cyclohexanon in Cyclohexan sowie 40 g Kobaltmolybdat in suspendierter Form 3 Stunden auf eine Tempertur von 80°C erhitzt. Nach gaschromatographischer Analyse erzielt man eine Ausbeute von 96 % Cyclohexenoxid.

## Patentansprüche

1. Verfahren zur Aufarbeitung von Cyclohexylhydroperoxid enthaltenden Reaktionsgemischen, die durch Oxidation von Cyclohexan mit molekularem Sauerstoff oder solchen enthaltenden Gasen in flüssiger Phase bei einer Temperatur von 130 bis 200°C und unter einem Druck von 5 bis 125 bar erhalten worden sind durch Umsetzen mit Cycloolefinen bei erhöhter Temperatur in Gegenwart von Katalysatoren, dadurch gekennzeichnet, daß man Cyclohexylhydroperoxid mit Cyclohexen bei erhöhter Temperatur in Gegenwart von in Cyclohexan löslichen Verbindungen von Übergangsmetallen der 4. bis 6. Gruppe des periodischen Systems oder mindestens einer in Cyclohexan unlöslichen Verbindung eines Übergangsmetalls der 4. bis 6. Gruppe, Selen, Tellur oder einem Borid umsetzt und dann das entstandene Cyclohexenoxid in Gegenwart von Hydrierkatalysatoren bei erhöhter Temperatur in an sich bekannter Weise zu Cyclohexanol hydriert.

2. Verfahren nach Anspruch 1, dadurch gekennzeichnet, daß man je Mol Cyclohexylhydroperoxid 1 bis 5 Mol Cyclohexen anwendet.

3. Verfahren nach den Ansprüchen 1 und 2, dadurch gekennzeichnet, daß man Gemische aus Cyclohexen und Cyclohexan, die durch partielle Hydrierung von Benzol erhalten worden sind, verwendet.

4. Verfahren nach den Ansprüchen 1 bis 3, dadurch gekennzeichnet, daß man bei der Umsetzung von Cyclohexylhydroperoxid mit Cyclohexen eine Temperatur von 50 bis 150°C einhält.

5. Verfahren nach den Ansprüchen 1 bis 4, dadurch gekennzeichnet, daß man in Cyclohexan lösliche Verbindungen der Metalle Titan, Vanadium, Molybdän oder Wolfram oder Gemische derselben oder in Cyclohexan unlösliche Verbindungen der Metalle Titan, Zirkon, Vanadium, Niob, Tantal, Chrom, Molybdän oder Wolfram als Katalysatoren verwendet.

6. Verfahren nach den Ansprüchen 1 bis 5, dadurch gekennzeichnet, daß man je Mol Cyclohexylhydroperoxid 0,0001 bis 0,001 Mol in Cyclohexan lösliche Verbindungen der Übergangsmetalle der 4. bis 6. Gruppe des periodischen Systems berechnet, als Metall anwendet.

7. Verfahren nach den Ansprüchen 1 bis 6, dadurch gekennzeichnet, daß man die Hydrierung von Cyclohexenoxid bei einer Temperatur von 80 bis 150°C durchführt.

8. Verfahren nach den Ansprüchen 1 bis 7, dadurch gekennzeichnet, daß man für die Hydrierung von Cyclohexenoxid Palladium auf Aktivkohleträger als Katalysator verwendet.

9. Verfahren nach den Ansprüchen 1 bis 8, dadurch gekennzeichnet, daß man ein Cyclohexanoxidationsgemisch verwendet, das durch partielles Abdestillieren von Cyclohexan angereichert wurde.

## Revendications

1. Procédé de traitement de mélanges réactionnels contenant de l'hydroperoxyde e cyclohexyle, qui ont été obtenus par oxydation du cyclohexane avec de l'oxygène moléculaire ou des gaz en contenant, en phase liquide à une température de 130 à 200°C et sous une pression de 5 à 125 bar, par réaction avec des cyclooléfines à température élevés en présence de catalyseurs, caractérisé en ce qu'on fait réagir l'hydroperoxyde de cyclohexyle avec du cyclohexène à température élevée en présence d'un composé soluble dans le cyclohexane d'un métal de transition des groupes IV à VI de la classification périodique ou d'au moins un composé insoluble dans le cyclohexane d'un métal de transition des groupes IV à VI, du sélénium, du tellure ou un borure, puis on hydrogène en cyclohexanol l'oxyde de cyclohexène obtenu, en présence de catalyseurs d'hydrogéneation à température élevée de façon connue en soi.

2. Procédé selon la revendication 1, caractérisé en ce qu'on utilise se 1 à 5 moles de cyclohexène par mole d'hydroperoxyde de cyclohexyle.

3. Procédé selon les revendications 1 et 2, caractérisé en ce qu'on utilise un mélange de cyclohexène et de cyclohexane qui a été obtenu par hydrogénation partielle de benzène.

4. Procédé selon les revendications 1 à 3, caractérisé en ce qu'on maintient une température de 50 à 150°C pendant la réaction de l'hydroperoxyde de cyclohexyle avec le cyclohexène.

5. Procédé selon les revendications 1 à 4, caractérisé en ce qu'on utilise comme catalyseurs des composés solubles dans le cyclohexane des métaux titane, vanadium, molybdène ou tungstène ou des mélanges de ceux-ci, ou des composés insolubles dans le cyclohexane des métaux titane, zirconium, vanadium, niobium, tantale, chrome, molybdène ou tungstène.

6. Procédé selon les revendications 1 à 5, caractérisé en ce qu'on utilise, par mole d'hydroperoxyde de cyclohexyle, de 0,0001 à 0,001 mole de composé soluble dans le cyclohexane d'un métal de transition des gorupes IV à VI de la classification périodique, exprimée en métal.

7. Procédé selon les revendications 1 à 6, caractérisé en ce qu'on mène l'hydrogénation de l'oxyde de cyclohexène à une température de 80 à 150°C.

8. Procédé selon les revendications 1 à 7, caractérisé en ce qu'on utilise comme catalyseur pour l'hydrogénation de l'oxyde de cyclohexène du palladium sur support de charbon actif.

9. Procédé selon les revendications 1 à 8, caractérisé en ce qu'on utilise un mélange d'oxydation du cyclohexane qui a été concentré par distillation partielle du cyclohexane.

## Claims

1. A process for working up a cyclohexyl hydroperoxide containing reaction mixture obtained by oxidizing cyclohexane with molecular oxygen or a molecular oxygen containing gas in liquid phase at from 130 to 200° and from 5 to 125 bar by reaction with a cycloolefin at elevated temperature in the presence of a catalyst, which comprises reacting cyclohexyl hydroperoxide with cyclohexene at elevated temperature in the presence of a cyclohexane-soluble compound of a transition metal of group 4 or 5 or 6 of the periodic table or one or more cyclohexane-insoluble compounds of a transition metal of group 4 or 5 or 6, or in the presence of selenium, tellurium or a boride, and then hydrogenating the resulting cyclohexene oxide in a conventional manner in the presence of a hydrogenation catalyst at elevated temperature to cyclohexanol.

2. A process as claimed in claim 1, wherein from 1 to 5 moles of cyclohexene are used per mole of cyclohexyl hydroperoxide.

3. A process as claimed in claim 1 or 2, wherein a mixture composed of cyclohexene and cyclohexane and obtained by partial hydrogenation of benzene is used.

4. A process as claimed in claim 1 or 2 or 3, wherein the reaction of cyclohexyl hydroperoxide with cyclohexene is carried out at from 5o to 150°C.

5. A process as claimed in claim 1 or 2 or 3 or 4, wherein the catalyst used is a cyclohexane-soluble compound of the metal titanium, vanadium, molybdenum or tungsten or a mixture thereof, or a cyclohexane-insoluble compound of the metal titanium, zirconium, vanadium, niobium, tantalum, chromium, molybdenem or tungsten.

6. A process as claimed in claim 1 or 2 or 3 or 4 or 5, wherein from 0.0001 to 0.001 mole of a cyclohexanesoluble compound of a transition metal of group 4 or 5 or 6 of the periodic table, calculated as metal, is used per mole of cyclohexyl hydroperoxide.

7. A process as claimed in claim 1 or 2 or 3 or 4 or 5 or 6, wherein the hydrogenation of cyclohexene oxide is carried out at from 80 to 150°C.

8. A process as claimed in claim 1 or 2 or 3 or 4 or 5 or 6 or 7, wherein the catalyst used for the hydrogenation of cyclohexene oxide is palladium on activated carbon.

9. A process as claimed in claim 1 or 2 or 3 or 4 or 5 or 6 or 7 or 8, wherein the cyclohexane oxidation mixture used has been enriched by partial distillative removal of cyclohexane.